# EUROPÄISCHE PATENTSCHRIFT

(11) **EP 2 571 547 B1**
(45) Veröffentlichungstag und Bekanntmachung des Hinweises auf die Patenterteilung: **27.03.2019**
(21) Anmeldenummer: 11720729.0
(22) Anmeldetag: 20.05.2011
(51) Int. Cl.: A61M 1/28

(54) **MEDIZINISCHE BEHANDLUNGSANORDNUNG**
MEDICAL TREATMENT ARRANGEMENT
SYSTÈME DE TRAITEMENT MÉDICAL

(30) Priorität: 20.05.2010 DE 102010022201
(43) Veröffentlichungstag der Anmeldung: 27.03.2013
(73) Patentinhaber: Fresenius Medical Care Deutschland GmbH, 61352 Bad Homburg (DE)
(72) Erfinder: BALSCHAT, Klaus, 97525 Schwebheim (DE); BREITKOPF, Berthold, 97424 Schweinfurt (DE); SAUER, Klaus, 97450 Reuchelheim (DE); HARTMANN, Marcus, 97421 Schweinfurt (DE); NIKOLIC, Dejan, 65812 Bad Soden (DE); HEIDE, Alexander, 65817 Eppstein (DE)
(74) Vertreter: Herrmann, Uwe
(86) Internationale Anmeldenummer: PCT/EP2011/002532
(87) Internationale Veröffentlichungsnummer: WO 2011/144355

(56) Entgegenhaltungen:
- EP-A1- 1 543 853
- EP-A2- 0 004 600
- WO-A1-2008/082033
- WO-A2-2004/062710
- WO-A2-2006/108026
- WO-A2-2007/048068
- CN-A- 1 859 936
- US-A- 5 762 782
- US-A1- 2007 135 779
- US-A1- 2008 230 450
- US-A1- 2010 204 765

## Beschreibung

Die vorliegende Erfindung betrifft eine medizinische Behandlungsanordnung medizinische Behandlungsanordnung mit wenigstens drei Geräteteilen, wobei es sich bei einem ersten Geräteteil um eine Befüll- und/oder Entleerstation handelt, die nicht zur Behandlung eines Patienten ausgebildet ist, wobei ein zweites Geräteteil über eine eigene Energieversorgung verfügt sowie mobil ausgeführt ist und Rollen zum Verfahren aufweist, sodass es relativ zu dem ersten Geräteteil und relativ zu einem dritten Geräteteil bewegbar ist, und wobei das dritte Geräteteil eine Behandlungsstation ist.

Aus dem Stand der Technik sind Dialysegeräte bekannt, bei denen die Dialysierflüssigkeit nicht während einer Behandlung hergestellt wird, sondern bei denen die gesamte für eine Dialysebehandlung erforderliche Menge an Dialysierflüssigkeit vor der Behandlung in einem Tank bereitgestellt wird. Derartige Dialysegeräte werden auch als "Batch-Type"-Dialysegeräte bezeichnet.

Um das Dialysegerät mit Behandlungsflüssigkeit zu befüllen, ist es denkbar, diese mittels einer mobilen Vorrichtung, die einen Tank oder ein sonstiges Behältnis aufweist, zu dem Dialysegerät zu verfahren und den Tankinhalt an das Dialysegerät zu übertragen, so dass die Behandlung des Patienten mit der Behandlungsflüssigkeit durchgeführt wird. Dabei ist es weiter denkbar, dass der Tank oder das sonstige Behältnis der mobilen Vorrichtung zuvor von einer Befüllstation mit der Behandlungsflüssigkeit befüllt wird.

Aus der US 2007/135779 A1 ist eine medizinische Behandlungsanordnung zur Sammlung und Beseitigung von verbrauchten medizinischen Flüssigkeiten wie beispielsweise Dialyseflüssigkeiten bekannt. Diese medizinische Behandlungsanordnung ist gemäß dem Oberbegriff des Anspruchs 1 ausgebildet.

Ausgehend von dieser bekannten Anordnung liegt der vorliegenden Erfindung die Aufgabe zugrunde, bei einer solchen, aus mehreren Geräteteilen bestehenden medizinischen Behandlungsanordnung auch Einsatzmöglichkeiten jenseits der bloßen Abfallsammlung und -beseitigung zu ermöglichen.

Diese Aufgabe wird durch eine medizinische Behandlungsanordnung mit den Merkmalen des Anspruchs 1 gelöst.

Dabei ist vorgesehen, dass das zweite Geräteteil in Verbindung mit dem dritten Geräteteil derart ausgebildet ist, dass mit diesen ein Dialyseverfahren durchführbar ist, und dass Beutel oder andere Behälter als Disposables in das zweite Geräteteil eingesetzt sind, und dass das erste und zweite Geräteteil und/oder das zweite und dritte Geräteteil derart ausgeführt sind, dass zwischen diesen Daten unidirektional oder bidirektional austauschbar sind, wobei das zweite Geräteteil einen Speicher aufweist, in dem Behandlungsdaten betreffend die Behandlung des Patienten und/oder Daten betreffend in das zweite Geräteteil und/oder in das dritte Geräteteil eingesetzte Beutel oder andere Behälter abgespeichert sind.

So handelt es sich bei dem zweiten Geräteteil um eine mobile Vorrichtung mit einem Tank in Form eines Beutels oder anderen Behälters als Disposable, mittels derer Behandlungsflüssigkeit zu dem dritten Geräteteil, das heißt zu dem Dialysegerät gebracht werden kann. Bei dem ersten Geräteteil, das nicht zur Behandlung des Patienten dient, handelt es sich um eine Befüll- und/oder Entleerstation (im Folgenden teilweise auch nur als Befüllstation bezeichnet), mittels derer der Tank des zweiten Geräteteils bei Bedarf befüllt oder entleert werden kann.

Erfindungsgemäß ist vorgesehen, dass zwischen diesen Geräteteilen ein Austausch von Daten und/oder von Energie möglich ist.

Dies hat den Vorteil, dass beispielsweise Informationen über die Befüllung des Tanks des zweiten Geräteteils beispielsweise in dem zweiten Geräteteil oder in dessen Tank gespeichert werden können und diese Daten dann von dem dritten Geräteteil, das heißt von dem Behandlungsgerät ausgelesen werden können. In diesem Fall dient das zweite Geräteteil als Datenüberträger zwischen erstem und drittem Geräteteil.

In einer bevorzugten Ausgestaltung der Erfindung ist vorgesehen, dass das erste und/oder das dritte Geräteteil immobil oder im wesentlichen immobil ausgebildet sind. Zwingend erforderlich ist dies jedoch nicht. Alternativ kann vorgesehen sein, dass das dritte Geräteteil gegebenenfalls auch bewegbar ausgeführt ist.

Das zweite Geräteteil ist mobil ausgeführt und weist Rollen auf, mittels derer es verfahrbar ist. Mittels dieser mobilen Vorrichtung, die durch einen Nutzer verfahrbar sein kann, kann die Überführung der Behandlungsflüssigkeit von einer Befüllstation zu dem Behandlungsgerät vorgenommen werden.

Parallel zu der Überführung dieser Behandlungsflüssigkeit ist es denkbar, dass zusätzlich Energie und/oder Daten übertragen werden, dass also das zweite Geräteteil als Energie- und/oder Datenspeicher bzw. -übertrager dient.

Gemäß der Erfindung ist vorgesehen, dass das zweite Geräteteil in Verbindung mit dem dritten Geräteteil derart ausgeführt sind, dass mit diesen ein Dialyseverfahren, insbesondere eine Hämodialyse oder Peritonealdialyse durchführbar ist.

In weiterer Ausgestaltung der Erfindung ist vorgesehen, dass wenigstens eines der Geräteteile, vorzugsweise mehrere oder alle Geräteteile der medizinischen Behandlungsanordnung derart ausgeführt sind, dass die Datenübertragung und/oder die Energieübertragung drahtlos oder drahtgebunden erfolgt.

Denkbar ist es beispielsweise, dass eine Datenübertragung und/oder Energieübertragung durch Induktion erfolgt. Ebenso ist es möglich, Daten und Energie durch eine mechanische Verbindung, beispielsweise durch ein Kabel oder eine elektrische Steckverbindung oder dergleichen zu übertragen. So ist es beispielsweise denkbar, dass der erste Geräteteil, das heißt beispielsweise die Befüllstation Energie drahtlos über Induktion oder über eine lösbare elektrische Verbindung an den zweiten Geräteteil überträgt bzw. sendet, der diese beispielsweise in einem Akkumulator oder einer entsprechenden anderen technischen Vorrichtung zum Speichern von Energie (Kondensatoren) speichert.

Denkbar ist es, dass wenigstens eines der Geräteteile derart ausgeführt ist, dass die Übertragung von Daten und Energie zeitgleich und/oder mittels derselben Einrichtung ausgetauscht werden. Denkbar ist es, dass ein Transpondersystem besonders bevorzugt so ausgelegt ist, das Daten und Energie zur gleichen Zeit über dasselbe System ausgetauscht werden. Dies geschieht in bevorzugter Ausgestaltung drahtlos und vorzugsweise induktiv.

Gemäß der Erfindung ist vorgesehen, dass das zweite Geräteteil über eine eigene Energieversorgung, insbesondere über einen Akkumulator und/oder über einen eigenen Speicher zur Speicherung von Daten verfügt.

Es kann vorgesehen sein, dass das zweite Geräteteil zur Speicherung von Daten über einen oder mehrere nicht flüchtige Speicher verfügt.

Das zweite Geräteteil kann Daten drahtlos beispielsweise über ein Transpondersystem an den ersten Geräteteil und/oder an den dritten Geräteteil senden und von diesem bzw. diesen auch drahtlos empfangen. Dies stellt eine bevorzugte Ausgestaltung der Erfindung dar. Anstelle oder zusätzlich zu einer drahtlosen Übertragung ist selbstverständlich auch eine drahtgebundene Übertragung denkbar.

So ist es beispielsweise denkbar, dass das zweite Geräteteil Energie drahtlos über Induktion oder über eine lösbare elektrische Verbindung an das erste und/oder an das dritte Geräteteil überträgt.

In einer beispielhaften Ausführungsform ist vorgesehen, dass eine eigenständige Energieversorgung des zweiten Geräteteils geladen wird und/oder dass Daten zwischen ersten, zweiten und dritten Geräteteil ausgetauscht werden, wobei vorzugsweise vorgesehen ist, dass der zweite Geräteteil als mobiler drahtloser Datenspeicher dient.

In weiterer Ausgestaltung der Erfindung ist vorgesehen, dass das zweite Geräteteil über Mittel verfügt, mittels derer im Falle eines Ausfalls der Energieversorgung des ersten und/oder dritten Geräteteils eine Notenergieversorgung des ersten und/oder dritten Geräteteils mittels des zweiten Geräteteils erfolgt. So ist es also denkbar, dass das zweite Geräteteil die zur Überbrückung von Stromausfällen dem ersten oder dritten Geräteteil, der beispielsweise als Befüllstation oder als Behandlungsgerät ausgeführt sein kann vorhandene eigene Energieversorgung unterstützt. Dies kann beispielsweise durch eine lösbare elektrische Verbindung (Stecker-Buchse), oder drahtlos über eine induktive Energieübertragung geschehen. Diese Situation kann bei längerfristigen Stromausfällen aufkommen. Denkbar ist beispielsweise auch eine Akutsituation, beispielsweise nach einem Unfall.

Gemäß der Erfindung ist vorgesehen, dass das zweite Geräteteil einen Speicher aufweist, in dem Behandlungsdaten betreffend die Behandlung des Patienten und/oder Daten betreffend in das zweite Geräteteil und/oder in das dritte Geräteteil eingesetzte Beutel oder andere Behälter abgespeichert sind. Somit ist es beispielsweise denkbar, dass das zweite Geräteteil von dem dritten Geräteteil Daten über Behandlungsparameter der bevorstehenden Behandlung erhält.

In weiterer Ausgestaltung der Erfindung ist vorgesehen, dass Mittel vorgesehen sind, die derart ausgeführt sind, dass in dem zweiten Geräteteil abgespeicherte Daten, wie beispielsweise Behandlungsdaten und/oder Daten betreffend eingelegte Disposables oder anderer Bestandteile der Geräteteile bei Annäherung oder Ankopplung an den ersten oder dritten Geräteteil an diesen übersandt werden oder auch dass eine Übersendung von Daten in die entgegengesetzte Richtung erfolgt. Die Daten können dann dort entweder direkt weiterverarbeitet werden oder beispielsweise über eine Netzwerkverbindung an ein zentrales Steuergerät, wie einen Zentralrechner, weitergeleitet werden.

So ist es beispielsweise denkbar, dass Daten bei der Annäherung des zweiten Geräteteils an den ersten Geräteteil, der beispielsweise als Befüll- und/oder Entleerstation ausgeführt sein kann, an letztere übergeben werden und dort entweder direkt weiterverarbeitet oder über die genannte Netzwerkverbindung an ein zentrales Steuergerät (Zentralrechner) weitergleitet werden.

In weiterer Ausgestaltung der Erfindung ist vorgesehen, dass Prüfmittel vorgesehen sind, die derart ausgeführt sind, dass die seitens des zweiten Geräteteils an das erste oder dritte Geräteteil übersandten Daten einer Prüfung unterzogen werden. Denkbar ist es beispielsweise, dass anhand der übertragenen Daten der erste Geräteteil, das vorzugsweise als Befüllstation ausgeführt ist, oder eine zentrale Steuereinheit über die Befüllung des Tanks bzw. Beutels (Menge und Zusammensetzung) in den zweiten Geräteteil entscheidet und bedarfsweise auf veränderte Behandlungsparameter an das zweite Geräteteil weitergibt, wenn z. B. ein Expertensystem in dem ersten Geräteteil oder in den damit verbundenen entfernten Steuergerät nach Bewertung der übergebenen Daten zu anderen Behandlungsparametern kommt.

In weiterer Ausgestaltung der Erfindung ist vorgesehen, dass die medizinische Behandlungsanordnung Entscheidungsmittel aufweist, die derart ausgeführt sind, dass anhand des Ergebnisses der in den Prüfmitteln vorgenommenen Prüfung eine Entscheidung darüber gefällt wird, ob eine Behandlung durchgeführt wird oder nicht. Somit ist es denkbar, dass beispielsweise der erste Geräteteil oder ein damit verbundenes vorzugsweise entferntes Steuergerät nach Bewertung der übergebenen Daten die Befüllung des Tankes des zweiten Geräteteils verweigert und somit eine Behandlung unmöglich macht, sollte sich herausstellen, dass beispielsweise ein ungeeignetes Disposable in dem zweiten Geräteteil oder auch in dem dritten Geräteteil eingelegt wurde.

Dies kann beispielsweise dann der Fall sein, wenn beispielsweise ein vormals schon benutzter Beutel oder Disposable verwendet werden, oder wenn Beutel oder Disposables, die ungeeignet sind, weil sie beispielsweise zu klein sind, falsch ausgeführt sind oder sonstige ungenügende Eigenschaften aufweisen, verwendet werden sollen.

In weiterer Ausgestaltung der Erfindung ist vorgesehen, dass in dem zweiten Geräteteil und/oder in dem dritten Geräteteil Disposables eingesetzt sind, die mit auslesbaren Markierungen oder dergleichen versehen sind.

Auf diese Weise ist es nicht nur denkbar, eine Identifikation der Disposables vorzunehmen, etwa dahingehend, um welche Größe und welchen einzufüllenden oder eingefüllten Inhalt es sich handelt, sondern auch, Fremdfabrikate bzw. das Fehlen von Merkmalen zu detektieren, die auf die Verwendung von Plagiaten oder Fremdfabrikaten hinweisen.

Der erste Geräteteil oder ein damit verbundenes Steuergerät, das entfernt angeordnet sein kann, kann in diesem Fall die Befüllung verweigern und dem zweiten Geräteteil ein Sperrsignal übermitteln, das dem dritten Geräteteil bei Annäherung oder Ankopplung des zweiten Geräteteils an das dritte Geräteteil signalisiert, dass die Behandlung nicht gestartet werden darf.

Bei diesen Markierungen kann es sich beispielsweise um zweidimensionale Matrizen oder um RFID-Tags handeln. Denkbar sind auch sämtliche weiteren geeigneten Markierungen, wie beispielsweise Barcodes.

Denkbar ist es somit, Originaldisposables mit fälschungssicheren Markierungen zu versehen, die sowohl Art als auch Exemplar kennzeichnen und den ersten Geräteteil, vorzugsweise der Befüllstation oder einen damit verbundenen entfernten Steuergerät bekannt gemacht werden. So kann erkannt werden, ob Originaldisposables zur Verwendung kommen und ob diese unbenutzt sind.

Darüber hinaus können auf diese Art auch Verfallsdaten abgeglichen werden, um die Verwendung zu lange gelagerter Disposables zu vermeiden. Wie ausgeführt, können die Markierungen in vielfältiger Form ausgeführt sein, wie beispielsweise Barcode, 2D, RF, etc.

Eine weitere Ausführungsform der vorliegenden Erfindung betrifft die verwechslungssichere Identität der einzelnen Geräteteile.

Bevorzugt ist es, dass das erste Geräteteil und/oder das zweite Geräteteil und/oder das dritte Geräteteil über Kennungen, vorzugsweise über elektronische Label, verfügen, mittels derer die jeweiligen Geräteteile selbst und/oder die Zugehörigkeit eines Geräteteils zu einem anderen Geräteteil identifizierbar ist. In dem eingangs genannten Ausführungsbeispiel ist es beispielsweise denkbar, dass die Behandlungsstation (drittes Geräteteil) der verfahrbare Trolley (zweites Geräteteil) und gegebenenfalls auch die Befüllstation (erstes Geräteteil) über eindeutige Merkmale verfügen, bevorzugt über elektronische Labels, die dem jeweils anderen Gerät bzw. Geräteteil bekannt gemacht werden. Auf diese Weise kann verhindert werden, dass beispielsweise ein befüllter Trolley (zweites Geräteteil) einer falschen Behandlungseinheit (drittes Geräteteil) zugeordnet wird.

Das elektronische Label oder auch eine beliebige andere geeignete Markierung kann ähnlich wie die Markierungen der Disposables ausgeführt sein. Bevorzugt senden aber die Trolleybefüll- und Behandlungsstationen bzw. die erste, zweite und/oder dritte Geräteteile bei der drahtlosen Kommunikation oder auch bei der drahtgebundenen Kommunikation individuelle Kenncodes, durch die eine eindeutige Zuordnung möglich ist.

Auf diese Weise ist es möglich, dass die Geräteteile zusammenwirken, für die dies auch vorgesehen ist.

In weiterer Ausgestaltung der Erfindung ist vorgesehen, dass das erste Geräteteil und/oder das zweite Geräteteil und/oder das dritte Geräteteil über ein oder mehrere Anzeige- und/oder Eingabeeinrichtungen, insbesondere Displays verfügt. Hinsichtlich der Art der Displays sind keine Beschränkungen vorhanden. Es kann sich beispielsweise um ein TFT (LCD), OLED oder E-Paper-Display handeln.

So ist es beispielsweise denkbar, dass das zweite Geräteteil, insbesondere ein verfahrbarer Trolley zum Zwecke der Informationsanzeige mit einem Display und/oder mit anderen optischen, akustischen oder haptischen Signalanzeigen versehen ist. Dies gilt für die anderen Geräteteile entsprechend.

Diese Signalanzeigen bzw. Displays können wie ausgeführt alle Arten von Displays umfassen. Ferner können Eingabevorrichtungen, wie beispielsweise eine Tastatur oder ein Touch-Screen vorgesehen sein. Dabei ist es denkbar, dass Touch-Screen und Anzeigeeinrichtung durch ein oder dasselbe Bauteil gebildet werden.

Handelt es sich um ein elektronisches Display (E-Paper) bietet dies den Vorteil, dass ein Trolley bzw. das zweite Geräteteil auch ohne interne Energiespeicherung ausgeführt werden kann, da die Anzeige des Displays auch ohne Stromversorgung aufrechterhalten wird. So kann der Bediener die Informationen auch dann ablesen, wenn der Trolley bzw. das zweite Geräteteil sich nicht in dem ersten oder dritten Geräteteil, das heißt gemäß einem bevorzugten Ausführungsbeispiel nicht in der Befüllstation- und/oder in der Behandlungseinheit befindet.

Die erfindungsgemäße Daten- und/oder Energieübertragung geschieht vorzugsweise induktiv, nach bekannten Verfahren. Hierzu verfügt der Sender über eine Sendespule, die ein magnetisches Wechselfeld ausstrahlt. Der Empfänger kann über eine Empfangsspule verfügen, in die eine elektrische Wechselspannung induziert wird, wenn sie sich im Feld der Sendespule befindet. Diese Wechselspannung wird am Empfänger gleichgerichtet und ausführungsabhängig die resultierende elektrische Energie vorzugsweise in einer Akkumulatorenbatterie gespeichert.

Die Datenübertragung kann hierbei auf vielfältige Weise geschehen. Beispielhaft, jedoch nicht einschränkend können hierbei genannt werden: Frequenzmodulation, Phasenmodulation, Amplitudenmodulation, Frequenzpulse. Vorzugsweise wird die Anordnung Sende-Empfängerspule durch Hinzufügen von blindleistungskompensierenden Bauteilen (Kapazitäten) in Resonanz zur Sendefrequenz betrieben, um eine möglichst gute Energieübertragung zu gewährleisten. Deshalb sind bei der Energieübertragung Modulationsarten bevorzugt, die die Frequenz des Sendesignals nicht ändern (Phasenmodulation, Frequenzpulse).

Die Datenübertragung vom Empfänger zum Sender kann in bekannter Art durch Lastmodulation erfolgen. Hierbei wird durch dem Dateninhalt entsprechendes Hinzufügen eines Lastwiderstandes empfangsseitig die Sendeleistung primärseitig moduliert.

Weitere Einzelheiten und Vorteile der vorliegenden Erfindung werden anhand eines in der Zeichnung dargestellten Ausführungsbeispiels näher erläutert.

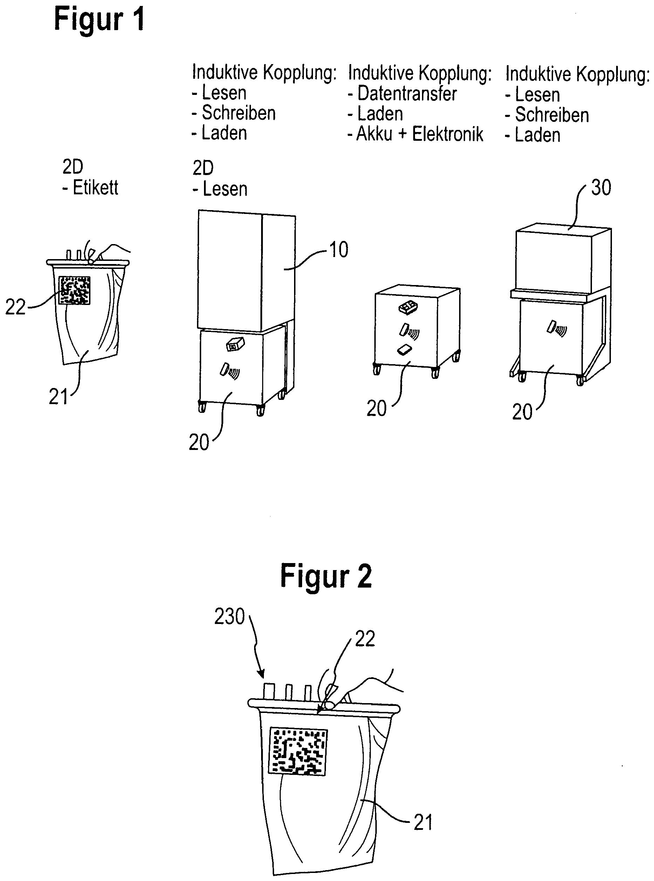

Die Ausführungsbeispiele betreffen drei Varianten der erfindungsgemäßen Behandlungsanordnung, wobei in der ersten Variante (Figur 1 bis Figur 16) das Disposable mit einer 2-D-Matrix ausgeführt ist und die Kommunikation zwischen Füllstation und Trolley und Trolley und Behandlungseinheit durch induktive Kopplung erfolgt.

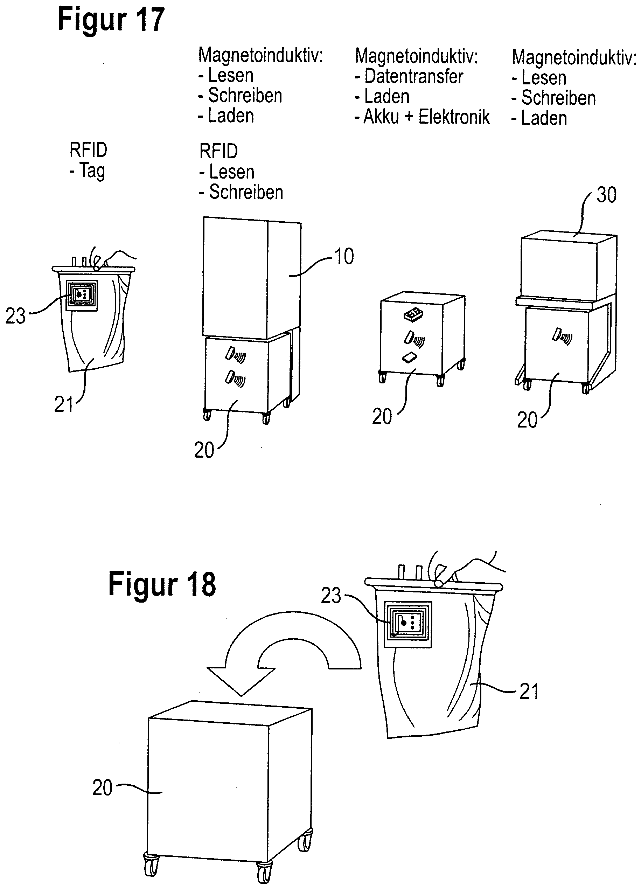

In der zweiten Variante gemäß der Figuren 17 bis 29 verfügt der Beutel über einen RFID-Tag und die Kommunikation zwischen Füllstation und Trolley und Trolley und Behandlungseinheit erfolgt magnetoinduktiv.

In der dritten Variante gemäß der Figuren 30 bis 43 verfügt der Beutel über eine 2D-Matrix und die Kommunikation zwischen Füllstation und Trolley und Trolley und Behandlungseinheit erfolgt mittels RFID.

Es zeigen:
- Figuren 1 bis 16:: den Daten- und Energietransfer zwischen Trolley, Befüllstation und Behandlungsgerät in einer ersten Ausführungsform,
- Figuren 17 bis 29:: den Daten- und Energietransfer zwischen Trolley, Befüllstation und Behandlungsgerät in einer zweiten Ausführungsform und
- Figuren 30 bis 43:: den Daten- und Energietransfer zwischen Trolley, Befüllstation und Behandlungsgerät in einer dritten Ausführungsform.

Figur 1 zeigt mit dem Bezugszeichen 10 eine als ersten Geräteteil bezeichneten Bestandteil der medizinischen Behandlungsanordnung in einem Ausführungsbeispiel der Erfindung. Es handelt sich dabei um eine Befüll- und Entleerstation zum Befüllen und Entleeren eines mobilen Trolleys 20, der als zweiter Geräteteil dient. Mit dem Bezugszeichen 30 ist der dritte Geräteteil dargestellt, der in dem hier dargestellten Ausführungsbeispiel als Dialysegerät zur Durchführung einer Dialysebehandlung ausgebildet ist.

In dem Trolley 20 befindet sich ein Beutel 21, der mit einem 2D-Etikett 22 ausgeführt ist, so dass Beutelart und -inhalt eindeutig identifizierbar sind. In dem hier dargestellten Ausführungsbeispiel erfolgt somit eine Datenübertragung vom Beutel an die Befüllstation über eine 2D-Matrix.

Sobald der Trolley 20 in die Befüllstation 10 eingeführt ist oder sich dieser genähert hat, kommt es zu einem Lese- bzw. Schreibvorgang, und zwar zwischen Trolley 20 und der Befüllstation 10 unidirektional oder auch bidirektional. Abgesehen davon kann es auch zu einem Ladevorgang, beispielsweise eines Akkumulators des Trolleys 20 durch die Befüllstation 10 kommen.

Der Trolley 20 kann außer einem Energiespeicher einen Datenspeicher und/oder eine Elektronik zur Auswertung von Daten aufweisen.

Der Energiespeicher kann beispielsweise induktiv von der Befüllstation 10 sowie auch von dem Behandlungsgerät 30 aufgeladen werden. Auch ist es denkbar, dass Energie von dem Energiespeicher des Trolleys 20 auf die Befüllstation 10 und/oder auf das Behandlungsgerät 30 übertragen wird.

Diese oben genannten Vorgänge können durch induktive Kopplung bzw. magnetoinduktiv erfolgen.

Entsprechendes gilt für die Kommunikation bzw. für den Energieaustausch zwischen Trolley 20 und dem Behandlungsgerät 30, wie dies ebenfalls in Figur 1 angedeutet ist.

Die Befüllstation 10 sowie das Behandlungsgerät 30 können vorzugsweise immobil oder im Wesentlichen immobil ausgeführt sein, jedenfalls für einen ortsfesten Einsatz gedacht sein. Grundsätzlich ist von der Erfindung jedoch auch der Fall erfaßt, dass auch diese Einheiten bewegbar sind, wie dies auch für den Trolley 20 gilt.

Figur 2 zeigt in vergrößerter Darstellung den Beutel 21 des Trolleys 20, der die Aufgabe hat, frische Dialysierlösungen von der Befüllstation 10 aufzunehmen und nach dem Verfahren zu dem Behandlungsgerät 30 die Lösung an dieses abzugeben bzw. diesem zur Verfügung zu stellen. Des Weiteren kann die Aufgabe darin bestehen, verbrauchte Dialyseflüssigkeit von dem Behandlungsgerät 30 aufzunehmen und dann nach dem Verfahren zur Befüllstation 10 an diese wieder abzugeben.

Insofern ist der Begriff "Befüllstation" weit zu fassen. Er kann sowohl den Befüllvorgang als auch den Entleerungsvorgang betreffen.

Mit dem Bezugszeichen 230 gemäß Figur 2 ist ein brechbarer Beutel-Pin gekennzeichnet. Dieser Pin wird beim Einlegen in das Behandlungsgerät 30 durch eine entsprechende Vorrichtung in dem Behandlungsgerät 30 erstmalig gebrochen und markiert damit einen gebrauchten Beutel. Ein solchermaßen markierter Beutel wird beim Wiedereinlegen in die Befüllstation durch einen entsprechenden Sensor (z. B. optisch oder mechanisch) erkannt und eine Wiederbefüllung verhindert. Auf diese Weise wird zuverlässig ein Re-Use verhindert.

In einem weiteren alternativen oder zusätzlich denkbaren Ausführungsbeispiel ist die Messung der Leitfähigkeit des Beutelinhaltes vorgesehen. Im unbenutzten Beutel können Konzentrate vorgehalten werden, die bei der Befüllung mit RO-Wasser in der Befüllstation 10 gelöst werden. Es stellt sich somit eine Lösung mit einer charakteristischen Leitfähigkeit ein, die sich durch die Benutzung in dem Behandlungsgerät 30 durch Stoffaustausch, beispielsweise während einer Dialysebehandlung, ändert. Die Befüllstation 10 prüft die Leitfähigkeit des befüllten Beutels und erkennt, wenn der Beutel nach Vorbenutzung nicht in der Befüllstation 10 entleert wurde und verbotener Weise zur Wiederbenutzung vorgesehen ist, dass keine Konzentrate, die die Leitfähigkeit charakteristisch verändern, im Beutel vorhanden waren. Somit wird auf Re-Use geschlossen und weitere Maßnahmen können ergriffen werden, um die Behandlung mit diesem wiederbenutzten Beutel zu verhindern. Solche Maßnahmen sind beispielsweise die Entleerung in der Befüllstation 10, das Erzeugen einer Warnanzeige oder eines sonstigen Alarmsignals, die Markierung des Beutels, etc.

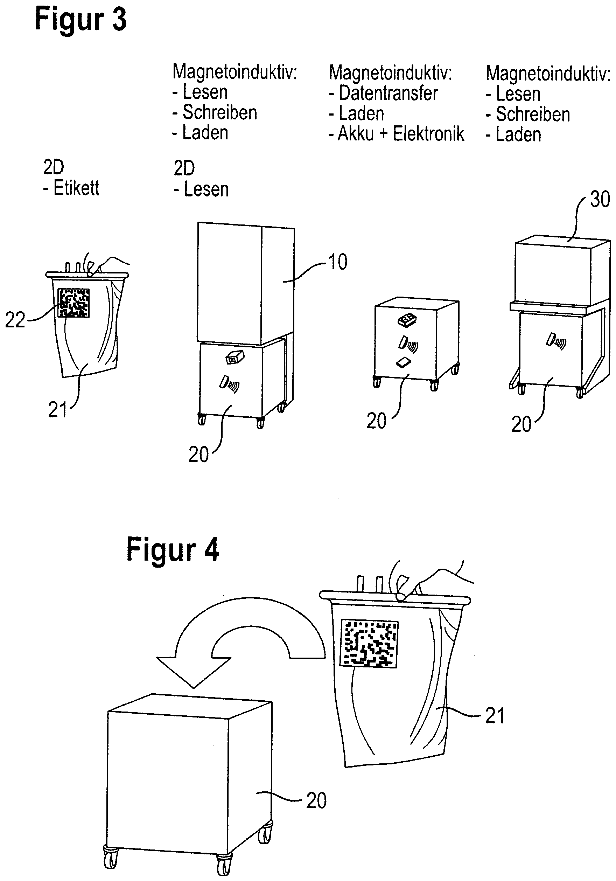

Figur 3 zeigt ein im Wesentlichen Figur 1 entsprechendes Übersichtsbild über die Komponenten der erfindungsgemäßen medizinischen Behandlungsanordnung, wobei hier klargestellt ist, dass in dem hier dargestellten Ausführungsbeispiel sowohl die Datenübertragung als auch die Übertragung von Energie magnetoinduktiv erfolgen.

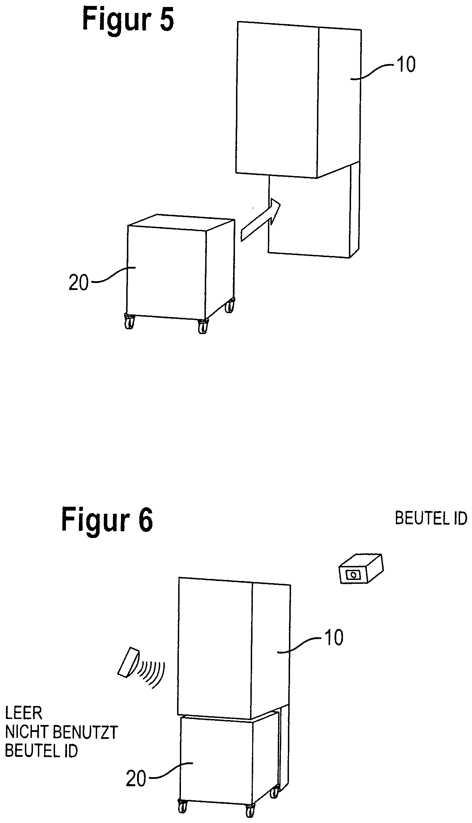

Figur 4 zeigt den Schritt des Aufrüstens des Trolleys 20 mit einem neuen Beutel 21. Wie dies aus Figur 5 hervorgeht, wird sodann der Trolley 20 mit der Befüllstation 10 konnektiert, und zwar derart, dass ein Fluidübergang und gegebenenfalls ein Daten- und/oder Energieübertrag möglich ist.

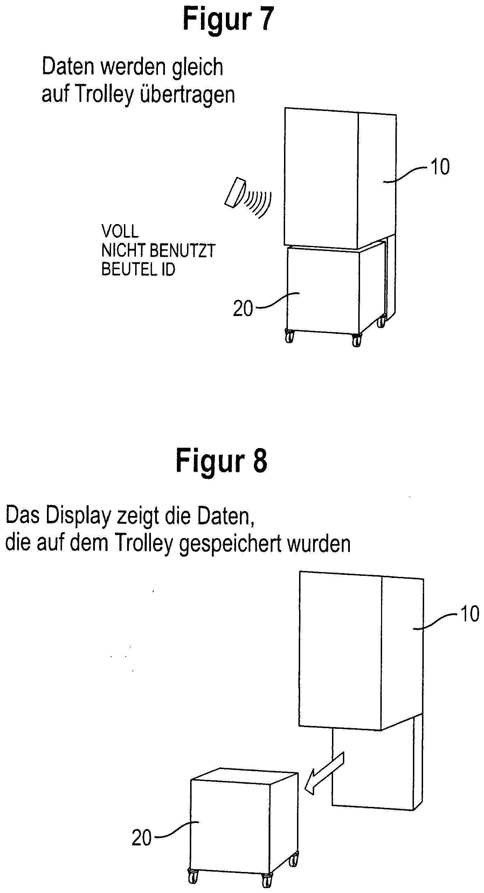

Figur 6 zeigt, dass die Beuteldaten und die Trolleydaten gelesen werden und Figur 7 zeigt den Zustand, dass der Beutel nunmehr gefüllt wurde und die Daten, insbesondere Daten zur Befüllung des Beutels auch gleich an den Trolley 20 übertragen werden. In dem dargestellten Beispiel wird an den Trolley 20 die Information übertragen, dass dessen Tank bzw. Beutel gefüllt ist und unbenutzte Dialyselösung enthält.

Grundsätzlich kann es sich bei den zwischen den Geräteteilen der vorliegenden Erfindung übertragenen Daten um beliebige Informationen, beispielsweise Behandlungsdaten, Patientendaten, Gerätedaten, Gerätekomponentendaten, etc. handeln. Denkbar ist es beispielsweise, dass die übertragene Information die Zusammensetzung der Konzentrate bzw. der fertigen Lösung betrifft.

Figur 8 zeigt den Zustand, in dem die Konnektion zwischen Trolley 20 und Befüllstation 10 getrennt wurde.

In bevorzugter Ausgestaltung der Erfindung ist vorgesehen, dass der Trolley 20 ein Display aufweist, in dem die Daten für den Nutzer sichtbar dargestellt werden, die in einer Speichereinheit des Trolleys gespeichert werden. Bei diesem Display kann es sich beispielsweise um ein E-Paper handeln, was den Vorteil mit sich bringt, dass ein Ablesen der Daten auch dann möglich ist, wenn der Trolley 20 über keine eigene Energieversorgung verfügt.

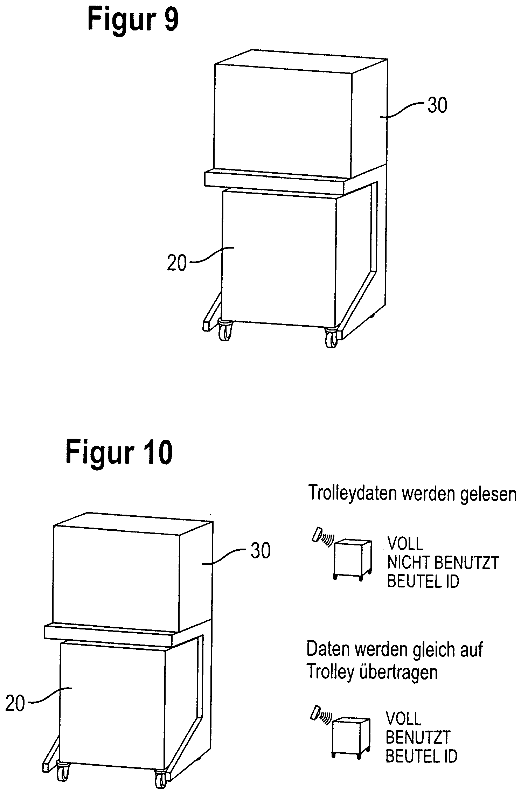

Der mittels der Befüllstation 10 befüllte Trolley 20 bzw. der befüllte Tank oder Beutel des Trolleys 20 wird sodann mit dem Trolley 20 zu dem Behandlungsgerät 30 verfahren und mit diesem konnektiert, wie dies aus Figur 9 hervorgeht.

Wie dies weiter aus Figur 10 hervorgeht, werden sodann die Trolleydaten ausgelesen und ebenfalls Daten auf den Trolley übertragen. Sofern die aus dem Trolley 20 ausgelesenen Daten in Ordnung sind, wird die Behandlung freigegeben.

In dem dargestellten Beispiel trägt der Trolley 20 nach Abschluß der Behandlung die Information, dass der Beutel voll ist und benutzt ist.

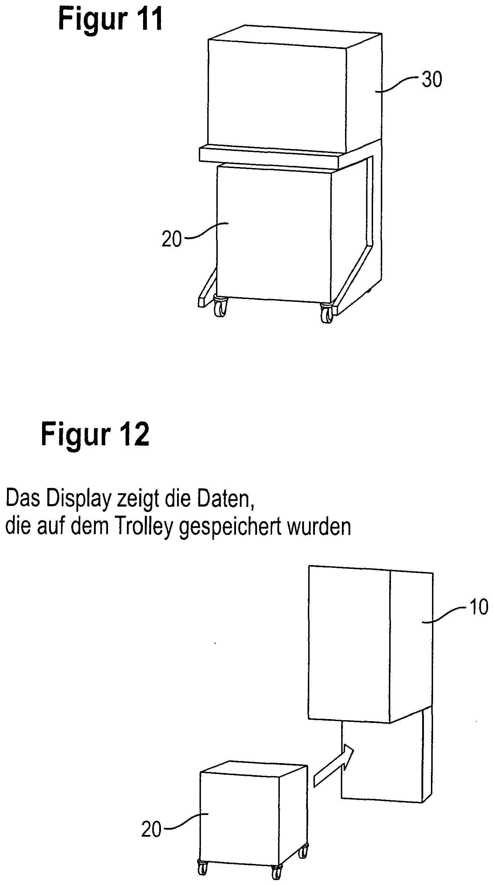

Figur 11 kennzeichnet das Ende der Behandlung und die Trennung der Konnektion zwischen Trolley 20 und Behandlungsgerät 30.

Das mit der benutzten Dialysierflüssigkeit gefüllte Behältnis des Trolleys 20 wird mit dem Trolley 20 sodann wieder zu der Befüllstation 10 verfahren, die in diesem Falle zur Entleerung des Tankes dient (vgl. Figur 12).

Dabei zeigt das Display des Trolleys 20 die Daten, die auf dem Trolley gespeichert wurden. Dabei kann es sich beispielsweise um die Daten handeln, die von dem Behandlungsgerät 30 auf den Trolley 20 übertragen wurden.

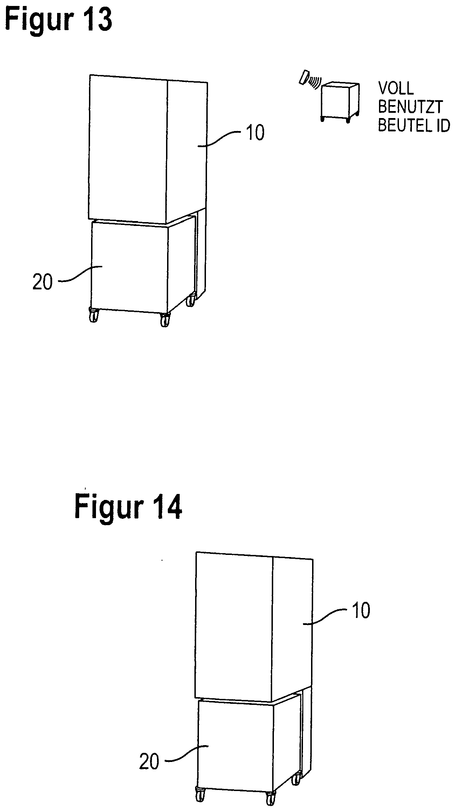

Figur 13 zeigt den Zustand, in dem die Trolleydaten von der Befüllstation 10 ausgelesen werden. Anschließend wird gemäß Figur 14 der Beutel des Trolleys 20 durch die Befüllstation 10 entleert.

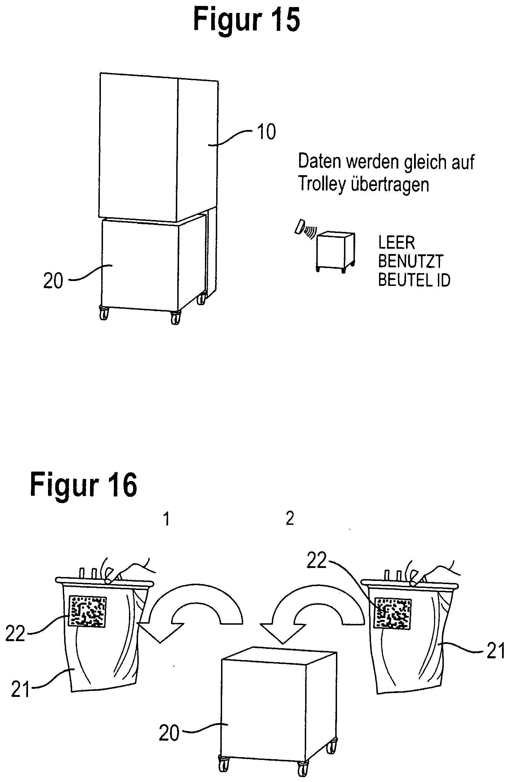

Figur 15 zeigt den Zustand, dass der Beutel entleert wurde und auch diese entsprechenden Daten werden sogleich auf den Trolley 20 übertragen. In diesem Fall handelt es sich um die Information, dass der Beutel leer und benutzt ist.

Anschließend schließt sich gemäß Figur 16 ein Beutelwechsel an, indem der benutzte Beutel gemäß Schritt 1 durch einen neuen Beutel gemäß Schritt 2 ersetzt wird.

Die Figuren 17 bis 29 zeigen ein weiteres Ausführungsbeispiel, das sich von dem ersten Ausführungsbeispiel gemäß den Figuren 1 bis 16 nur dadurch unterscheidet, dass der Beutel nicht mit einer 2D-Matrix als Kennzeichnungsmittel, sondern mit einem RFID-Tag ausgeführt ist, der auslesbar und beschreibbar ist. Im Übrigen wird auf die obigen Ausführungen zu dem ersten Ausführungsbeispiel entsprechend Bezug genommen.

In diesem Ausführungsbeispiel erfolgt die Kommunikation zwischen Befüllstation und Trolley sowie auch zwischen Trolley und Behandlungseinheit magnetoinduktiv, wie dies aus Figur 17 hervorgeht.

Figur 18 zeigt den Schritt des Aufrüstens des Trolleys 20 mit dem neuen Beutel 21, der als Kennzeichnungsmittel einen RFID-Tag 23 aufweist.

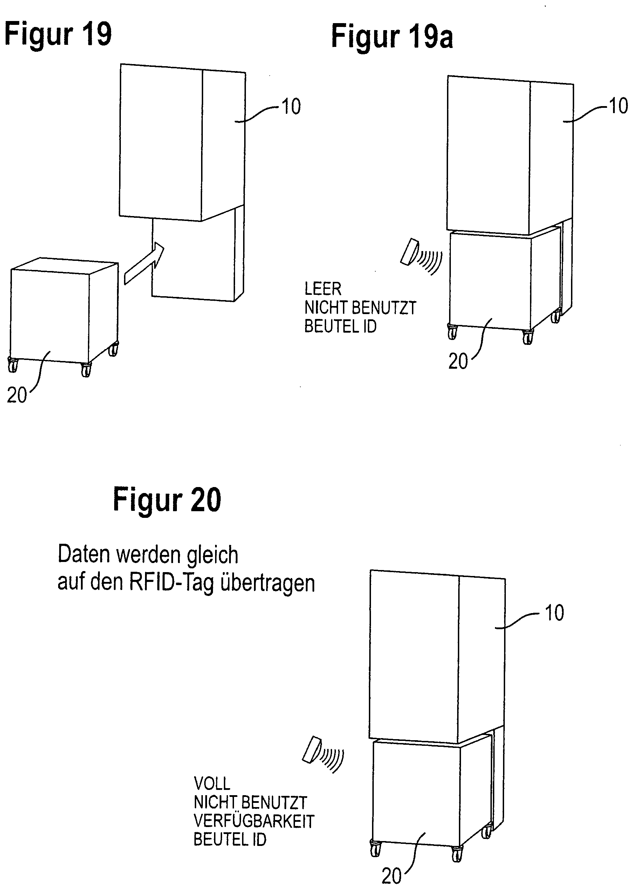

Figur 19 zeigt den Schritt der Konnektion des Trolleys 20, in den der Beutel 21 eingesetzt ist, mit der Befüllstation 10.

Gemäß Figur 19a werden sodann die Beuteldaten des Beutels des Trolleys 20 durch die Befüllstation 10 ausgelesen. In diesem Fall handelt es sich um die Beutel-Kennung sowie um die Information, dass der Beutel leer und unbenutzt ist.

Der Beutel wird danach gemäß Figur 20 durch die Befüllstation 10 gefüllt und die entsprechenden Daten gleich an den RFID-Tag des Beutels übertragen.

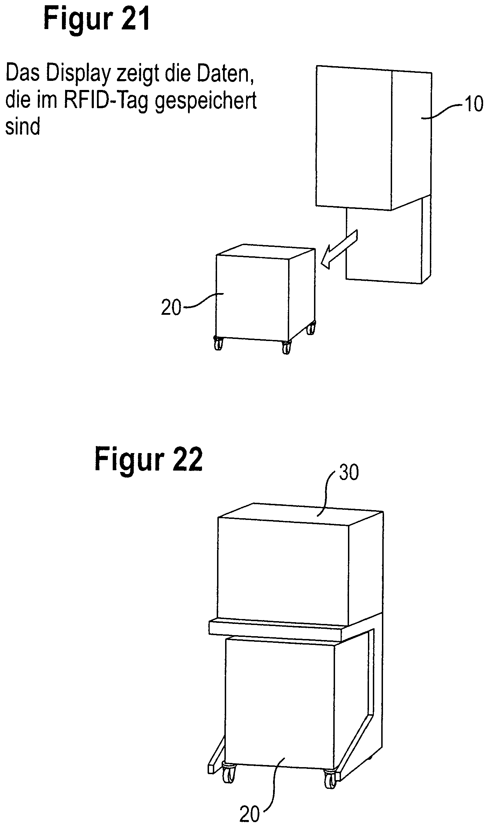

Gemäß Figur 21 wird sodann die Konnektion zwischen Trolley 20 und Befüllstation 10 getrennt und das Display des Trolleys 20 zeigt die Daten an oder jedenfalls einen Teil der Daten, die in dem RFID-Tag des Beutels gespeichert sind.

Figur 22 zeigt die Konnektion des Trolleys mit befülltem Tank mit dem Behandlungsgerät 20.

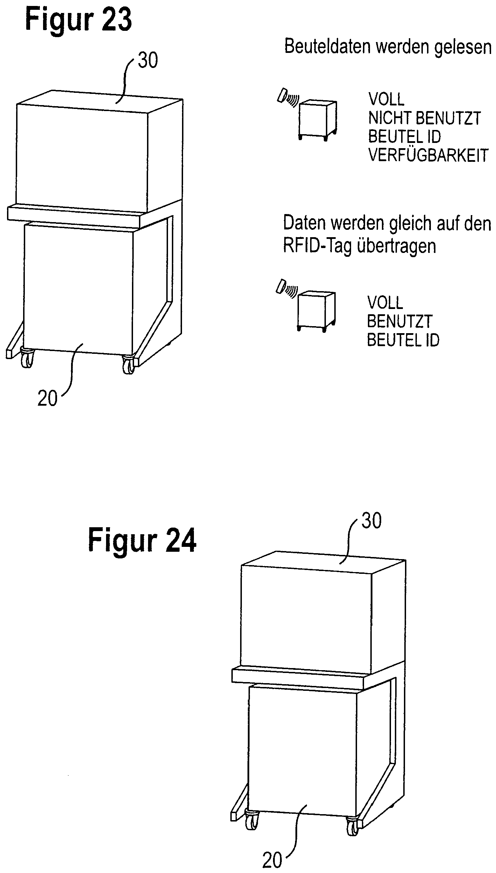

Figur 23 betrifft die Freigabe der Behandlung. Hierzu werden die Beuteldaten ausgelesen und die nach Durchführung der Behandlung vorliegenden Daten werden von dem Behandlungsgerät 30 auf den RFID-Tag des Beutels übertragen.

Figur 24 kennzeichnet das Ende der Behandlung und die Konnektion zwischen Trolley 20 und dem Behandlungsgerät 30 wird getrennt.

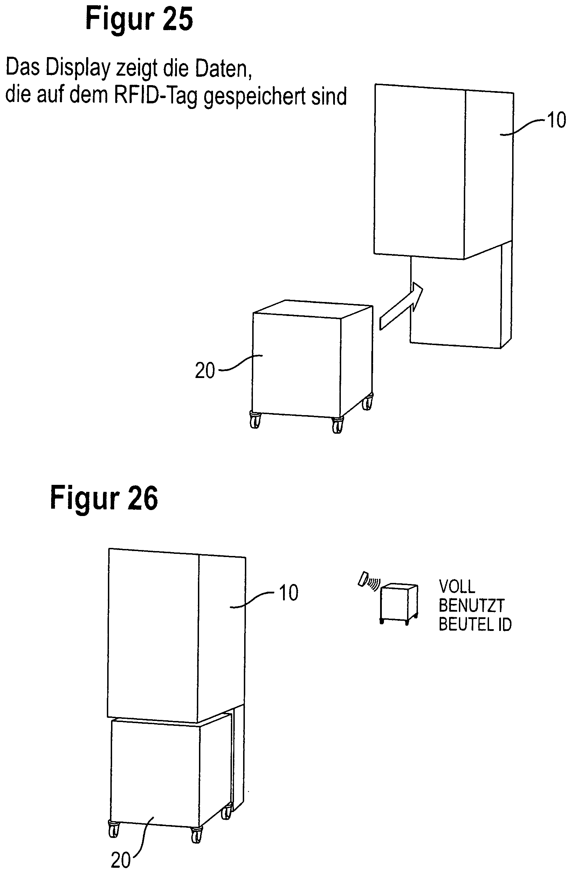

Der mit der benutzten Dialyselösung gefüllte Trolley 20 wird sodann zu der Befüllstation 10 verfahren (vgl. Figur 25). Der Trolley 20 zeigt in seinem Display die Daten an, die auf dem RFID-Tag gespeichert sind.

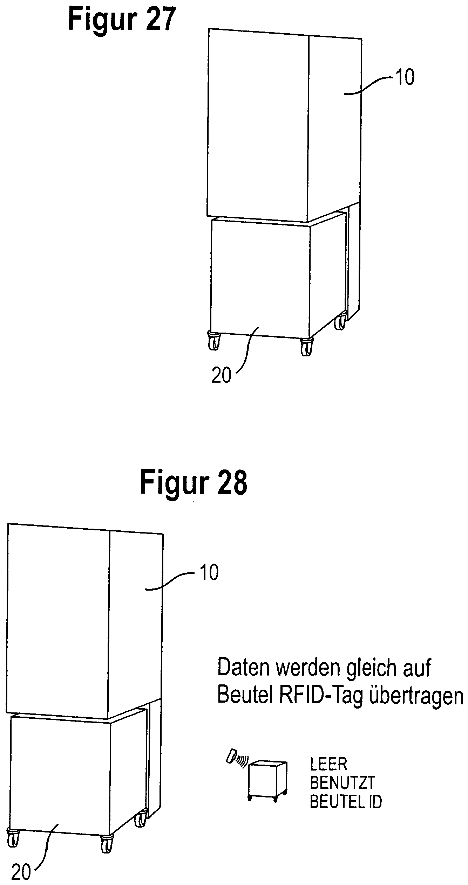

Figur 26 zeigt das Auslesen der Beuteldaten durch die Befüllstation 10 und Figur 27 zeigt den Vorgang der Beutelentleerung durch die Befüllstation 10.

Figur 28 zeigt einen Zustand, in dem der Beutel entleert wurde und die Daten gleich auf den Beutel RFID-Tag übertragen werden.

Daran schließlich sich wie auch in dem ersten Ausführungsbeispiel der Beutelwechsel gemäß der Schritte 1 und 2 an, wobei gemäß Schritt 1 der benutzte Beutel 1 gegen einen frischen Beutel 2 ausgetauscht wird.

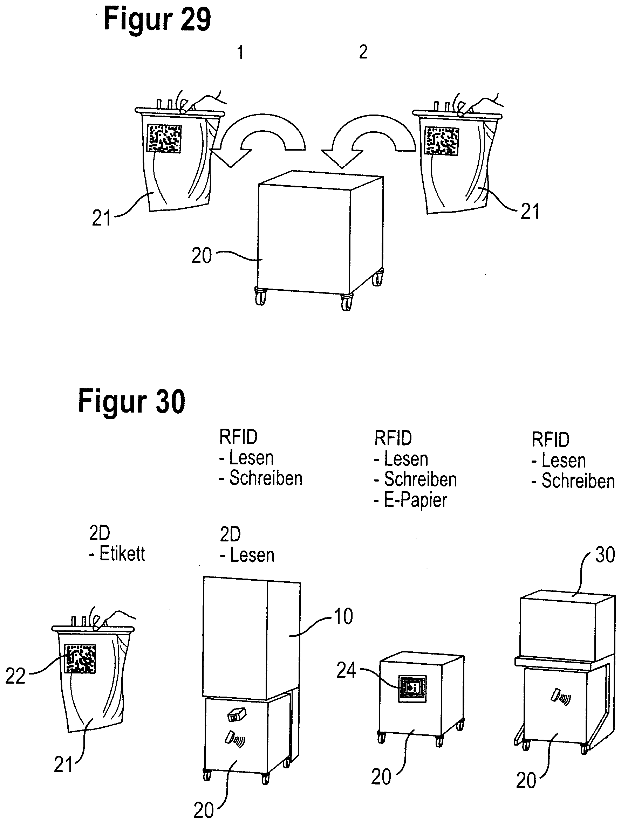

Die Figuren 30 bis 43 zeigen ein drittes Ausführungsbeispiel der vorliegenden Erfindung, bei dem der Beutel zu seiner Identifikation über eine 2D-Matrix verfügt und die Kommunikation zwischen Füllstation und Trolley und zwischen Trolley und Behandlungseinheit über RFID erfolgt.

Im Gegensatz zu den beiden ersten Ausführungsbeispielen ist in dem dritten Ausführungsbeispiel vorgesehen, dass der Trolley 20 in dieser Ausführungsform keinen eigenen Energiespeicher aufweist und die Anzeige von gespeicherten Daten über ein E-Paper-Display erfolgt. Grundsätzlich ist jedoch auch in diesem Ausführungsbeispiel ein Einsatz eines Trolleys mit eigenem Energiespeicher denkbar. Zur Beschreibung eines E-Papers und zum Beschreiben/Lesen von nichtflüchtigen Speichern (z. B. EEPROM) wird einmalig Energie benötigt. Ist der Vorgang der Zustandsänderung des Displays oder des oder der Speicher beendet, ist keine weitere Energie mehr notwendig, um den Zustand stabil zu halten. Wesentlich für das dritte Ausführungsbeispiel ist es, dass der Trolley 20 in dieser Ausführungsform keinen eigenen Energiespeicher benötigt.

Der Trolley 20 ist in diesem Ausführungsbeispiel mit einem RFID-Tag 24 ausgeführt, das lesbar und beschreibbar ist. Daten des RFID-Tags sind auf dem Display des Trolleys 20 darstellbar.

Aus Figur 30 ergibt sich, dass als Kommunikationsmedium zur Kommunikation zwischen Füllstation und Trolley und Trolley und Behandlungseinheit RFID verwendet wird und dass der Beutel 21 selbst mit einem 2D-Etticket bzw. mit einer 2D-Matrix ausgeführt ist.

Ansonsten entsprechen die Geräteteile dem Ausführungsbeispiel gemäß Figur 1, so dass entsprechend Bezug genommen wird.

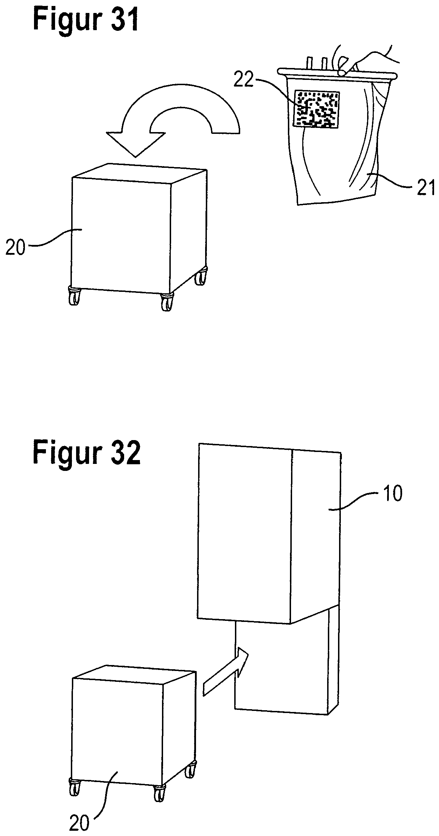

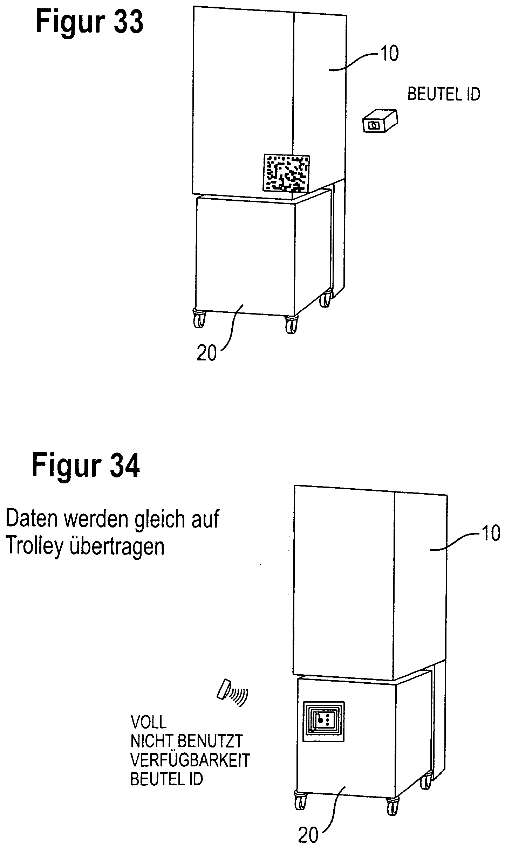

Figur 31 zeigt wieder das Aufrüsten des Trolleys 20 mit einem neuen Beutel 21, Figur 32 die Konnektion mit der Befüllstation 10 und Figur 33 das Auslesen der Beuteldaten des 2D-Ettickets, durch die Befüllstation 10.

Figur 34 zeigt das Befüllen des Beutels des Trolleys 20 durch die Befüllstation 10. Dabei werden Daten gleich auf den Trolley 20 bzw. auf dessen Speicher übertragen.

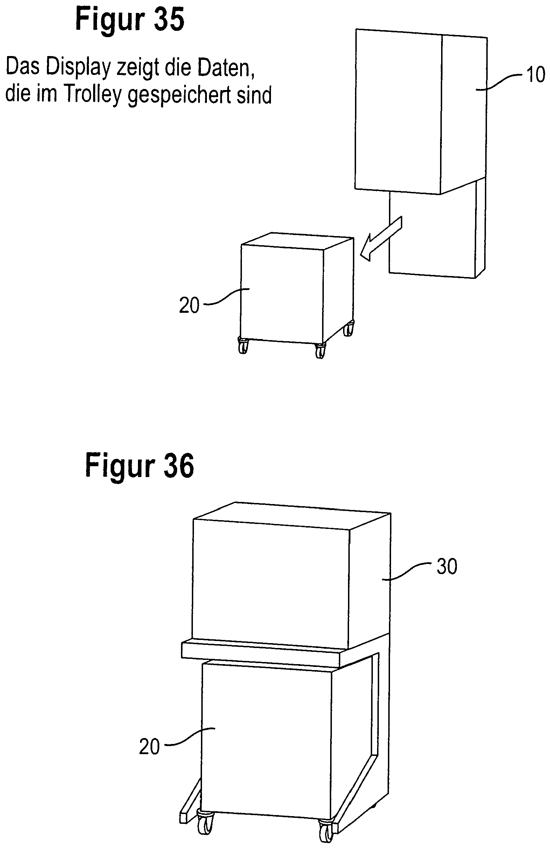

Gemäß Figur 35 wird die Konnektion dann getrennt und das Trolley zeigt auf einem als E-Paper ausgeführten Display die in dem Trolley 20 gespeicherten Daten.

Anschließend wird der Trolley 20 zu dem Behandlungsgerät 30 verfahren und mit diesem konnektiert, wie dies aus Figur 36 hervorgeht.

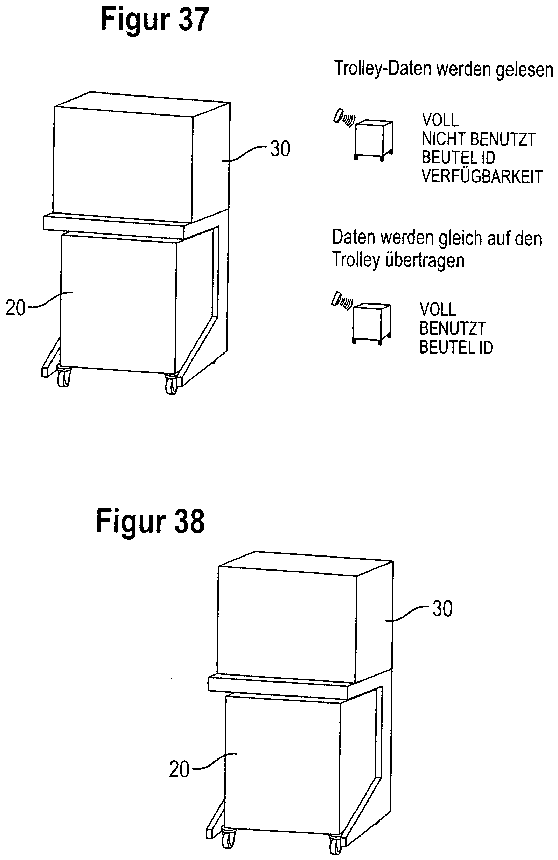

Sind die Trolleydaten in Ordnung, wird die Behandlung freigegeben. Die die Behandlung betreffenden Daten werden dann nach der Behandlung gleich auf den Trolley übertragen, wie dies aus Figur 37 hervorgeht.

Figur 38 kennzeichnet das Ende der Behandlung. Die Konnektion zwischen Behandlungsgerät 30 und Trolley 20 wird getrennt.

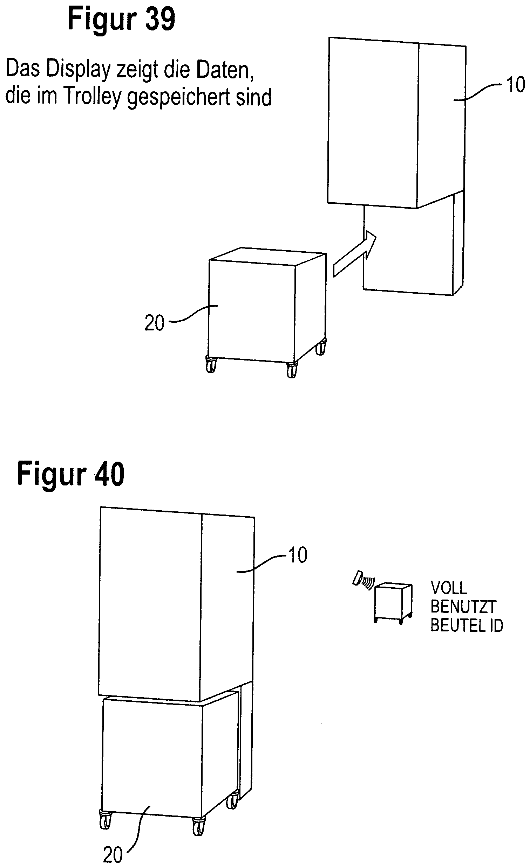

Figur 39 betrifft die Konnektion des Trolleys 20 mit der Füllstation 10. Auch hier zeigt das Display die Daten an, die nun im Trolley 20 gespeichert sind.

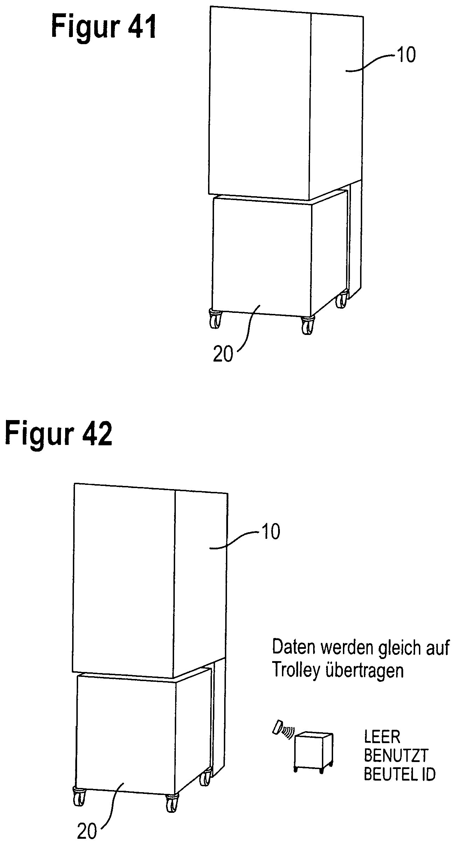

Gemäß Figur 40 werden dann die Trolley-Daten mittels der Befüllstation 10 ausgelesen und gemäß Figur 41 wird der Beutel entleert.

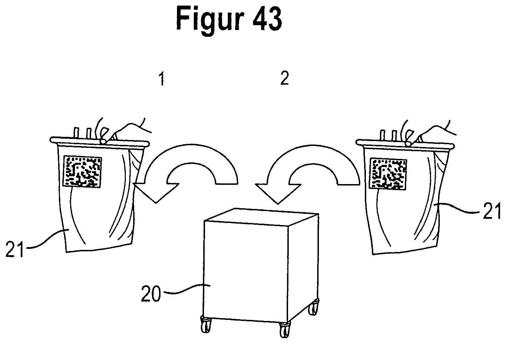

Die entsprechenden Daten werden dann nach der Entleerung des Tanks bzw. Beutels des Trolleys gleich auf den Trolley 20 gemäß Figur 42 übertragen, sobald der Beutel entleert wurde. Daran schließt sich gemäß Figur 43 wie auch in den vorausgegangenen Ausführungsbeispielen der Beutelwechsel an. Ein verbrauchter Beutel wird gemäß Schritt 1 durch einen frischen Beutel gemäß Schritt 2 ausgetauscht und der Trolley steht erneut zur Befüllung in einer Befüllstation zur Verfügung.

## Patentansprüche

1. Medizinische Behandlungsanordnung mit wenigstens drei Geräteteilen, wobei es sich bei einem ersten Geräteteil (10) um eine Befüll- und/oder Entleerstation handelt, die nicht zur Behandlung eines Patienten ausgebildet ist, wobei ein zweites Geräteteil (20) über eine eigene Energieversorgung verfügt sowie mobil ausgeführt ist und Rollen zum Verfahren aufweist, sodass es relativ zu dem ersten Geräteteil (10) und relativ zu einem dritten Geräteteil (30) bewegbar ist, und wobei das dritte Geräteteil eine Behandlungsstation ist,
**dadurch gekennzeichnet,**
**dass** das zweite Geräteteil (20) in Verbindung mit dem dritten Geräteteil (30) derart ausgebildet ist, dass mit diesen ein Dialyseverfahren durchführbar ist, dass Beutel oder andere Behälter als Disposables in das zweite Geräteteil (20) eingesetzt sind, und dass das erste (10) und zweite Geräteteil (20) und/oder das zweite (20) und dritte Geräteteil (30) derart ausgeführt sind, dass zwischen diesen Daten unidirektional oder bidirektional austauschbar sind, wobei das zweite Geräteteil (20) einen Speicher aufweist, in dem Behandlungsdaten betreffend die Behandlung des Patienten und/oder Daten betreffend in das zweite Geräteteil (20) und/oder in das dritte Geräteteil (30) eingesetzte Beutel oder andere Behälter abgespeichert sind.

2. Medizinische Behandlungsanordnung nach Anspruch 1, **dadurch gekennzeichnet, dass** das erste (10) und/oder das dritte Geräteteil (30) immobil oder im wesentlichen immobil ausgebildet sind.

3. Medizinische Behandlungsanordnung nach Anspruch 1 oder 2, **dadurch gekennzeichnet, dass** es sich bei dem Dialyseverfahren um eine Hämodialyse oder Peritonealdialyse handelt.

4. Medizinische Behandlungsanordnung nach einem der vorhergehenden Ansprüchen, **dadurch gekennzeichnet, dass** wenigstens eines der Geräteteile, vorzugsweise mehrere oder alle Geräteteile der medizinischen Behandlungsanordnung derart ausgeführt sind, dass die Datenübertragung und/oder eine Energieübertragung zwischen zwei Geräteteilen drahtlos oder drahtgebunden erfolgt.

5. Medizinische Behandlungsanordnung nach Anspruch 4, **dadurch gekennzeichnet, dass** wenigstens eines der Geräteteile, vorzugsweise mehrere oder alle Geräteteile derart ausgeführt sind, dass die Übertragung von Daten und Energie zeitgleich und/oder mittels derselben Einrichtung erfolgt.

6. Medizinische Behandlungsanordnung nach einem der vorhergehenden Ansprüche, **dadurch gekennzeichnet, dass** das zweite Geräteteil (20) zur Speicherung von Daten über einen oder mehrere nicht flüchtige Speicher verfügt.

7. Medizinische Behandlungsanordnung nach einem der vorhergehenden Ansprüche, **dadurch gekennzeichnet, dass** die Energieversorgung des zweiten Geräteteils (20) so ausgebildet ist, dass mittels ihr im Falle eines Ausfalls der Energieversorgung des ersten (10) oder dritten Geräteteils (30) eine Notenergieversorgung des ersten (10) oder dritten Geräteteils (30) mittels des zweiten Geräteteils (20) erfolgen kann.

8. Medizinische Behandlungsanordnung nach einem der vorhergehenden Ansprüche, **dadurch gekennzeichnet, dass** Mittel vorgesehen sind, die derart ausgeführt sind, dass in dem zweiten Geräteteil (20) abgespeicherte Daten bei Annäherung an den ersten (10) und/oder dritten Geräteteil (30) an diesen übersandt oder von diesem empfangen werden.

9. Medizinische Behandlungsanordnung nach einem der vorhergehenden Ansprüche, **dadurch gekennzeichnet, dass** Prüfmittel vorgesehen sind, die derart ausgeführt sind, dass die seitens des zweiten Geräteteils (20) an das erste (10) oder dritte Geräteteil (30) übersandten Daten einer Prüfung unterzogen werden, wobei vorzugsweise vorgesehen ist, dass Entscheidungsmittel vorgesehen sind, die derart ausgeführt sind, dass anhand des Ergebnisses der in den Prüfmitteln vorgenommenen Prüfung eine Entscheidung darüber gefällt wird, ob eine Behandlung durchgeführt wird oder nicht.

10. Medizinische Behandlungsanordnung nach einem der vorhergehenden Ansprüche, **dadurch gekennzeichnet, dass** die Disposables mit auslesbaren Markierungen versehen sind, wobei vorzugsweise vorgesehen ist, dass es sich bei der Markierung um eine 2D-Matrix, einen Barcode oder um ein RFID-Tag handelt.

11. Medizinische Behandlungsanordnung nach einem der vorhergehenden Ansprüche, **dadurch gekennzeichnet, dass** das erste Geräteteil (10) und/oder das zweite Geräteteil (20) und/oder das dritte Geräteteil (30) über Kennungen, vorzugsweise über elektronische Label, verfügen, mittels derer die jeweiligen Geräteteile selbst und/oder die Zugehörigkeit eines Geräteteils zu einem anderen Geräteteil identifizierbar ist.

12. Medizinische Behandlungsanordnung nach einem der vorhergehenden Ansprüche, **dadurch gekennzeichnet, dass** das erste Geräteteil (10) und/oder das zweite Geräteteil (20) und/oder das dritte Geräteteil (30) über ein oder mehrere Anzeige- und/oder Eingabeeinrichtungen, insbesondere Displays verfügt, wobei vorzugsweise vorgesehen ist, dass es sich bei dem Display um ein TFT (LCD), OLED oder E-Paper-Display handelt.

13. Medizinische Behandlungsanordnung nach einem der vorhergehenden Ansprüche, **dadurch gekennzeichnet, dass** das zweite Geräteteil (20) über wenigstens einen Tank zur Aufnahme einer frischen und/oder einer gebrauchten Behandlungsflüssigkeit und/oder zur Aufnahme von Substitutionsflüssigkeit verfügt.

14. Medizinische Behandlungsanordnung nach Anspruch 13, **dadurch gekennzeichnet, dass** der erste Geräteteil (10) und/oder der dritte Geräteteil (30) über Mittel verfügt, mittels derer der Tank des zweiten Geräteteils (20) befüllbar und/oder entleerbar ist.

## Claims

1. A medical treatment arrangement having at least three device parts, with the first device part (10) being a filling or emptying station which is not made for the treatment of a patient, with a second device part (20) having its own proper energy supply and being configured movably and comprising rollers for displacement, so that it is movable relative to the first device part (10) and relative to a third device part (30), and wherein the third device part is a treatment station,
**characterized in that**
the second device part (20) is made in conjunction with the third device part (30) that a dialysis process can be carried out with them, that bags or other containers are inserted in the second device part (20) as disposables, and that the first (10) and the second device part (20) and/or the second (20) and third device part (30) are configured such that data can be unidirectionally or bidirectionally exchanged between them, wherein the second device part (20) comprises a memory in which treatment data relating to the treatment of the patient and/or data relating to bags or other containers inserted into the second device part (20) and/or into the third device part (30) is stored.

2. A medical treatment arrangement in accordance with Claim 1, **characterized in that** the first device part (10) and/or the third device part (30) are made as immobile or as substantially immobile.

3. A medical treatment arrangement in accordance with Claim 1 or 2, **characterized in that** the dialysis process is preferably a hemodialysis or a peritoneal dialysis.

4. A medical treatment arrangement according to one of the preceding claims, **characterized in that** at least one of the device parts, preferably a plurality of device parts or all device parts, of the medical treatment arrangement are made such that the data transfer and/or an energy transfer between two device parts takes place in a wireless or wired manner.

5. A medical treatment arrangement in accordance with claim 4, **characterized in that** at least one of the device parts, preferably a plurality of the device parts or all device parts, are made such that the transfer of data and energy takes place at the same time and/or by means of the same device.

6. A medical treatment arrangement according to one of the preceding claims, **characterized in that** the second device part (20) has one or more non-volatile memories for the storage of data.

7. A medical treatment arrangement according to one of the preceding claims, **characterized in that** the energy supply of the second device part (20) is configured such that an emergency energy supply of the first device part (10) or of the third device part (30) can take place by means of the second device part (20) in the case of a failure of the energy supply of the first device part (10) or of the third device part (30).

8. A medical treatment arrangement in accordance with one of the preceding claims, **characterized in that** means are provided which are made such that data stored in the second device part (20) are transmitted to the first device part (10) and/or to the third device part (30) or are received by them when they are approached.

9. A medical treatment arrangement in accordance with one of the preceding claims, **characterized in that** test means are provided which are made such that the data transmitted by the second device part (20) to the first device part (10) or to the third device part (30) are subjected to a test, wherein it is preferably provided that decision means are provided which are made such that a decision is made with reference to the result of the test carried out in the test means as to whether a treatment is carried out or not.

10. A medical treatment arrangement in accordance with one of the preceding claims, **characterized in that** the disposables are provided with readable markings or the like, wherein it is preferably provided that the marking is a 2D matrix, a barcode or an RFID tag.

11. A medical treatment arrangement in accordance with one of the preceding claims, **characterized in that** the first device part (10) and/or the second device part (20) and/or the third device part (30) has/have marks, preferably electronic labels, by means of which the respective device parts themselves and/or the belonging of a device part to another device part can be identified.

12. A medical treatment arrangement in accordance with one of the preceding claims, **characterized in that** the first device part (10) and/or the second device part (20) and/or the third device part (30) has/have one or more indication devices and/or input devices, in particular displays, wherein it is preferably provided that the display is a TFT (LCD), OLED or e-paper display.

13. A medical treatment arrangement in accordance with one of the preceding claims, **characterized in that** the second device part (20) has at least one tank for the reception of a fresh treatment fluid and/or of a used treatment fluid and/or for the reception of substitution fluid.

14. A medical treatment arrangement in accordance with claim 13, **characterized in that** the first device part (10) and/or the third device part (30) has/have means by means of which the tank of the second device part (20) can be filled and/or emptied.

## Revendications

1. Système de traitement médical comportant au moins trois éléments d'appareil, le premier élément d'appareil (10) étant une station de remplissage et/ou de vidage qui n'est pas conçue pour le traitement d'un patient, le deuxième élément d'appareil (20) possédant sa propre source d'approvisionnement énergétique et est conçue de manière mobile et comporte des roulettes de manière à pouvoir être déplacé par rapport au premier élément d'appareil (10) et par rapport à un troisième élément d'appareil (30), et le troisième élément d'appareil étant une station de traitement,
**caractérisé en ce que**
le deuxième élément d'appareil (20) est conçu en liaison avec le troisième élément d'appareil (30) de telle sorte qu'il est possible de procéder avec ceux-ci à un procédé de dialyse, **en ce que** des poche ou d'autres récipients sont placés sous forme de produits jetable dans le deuxième élément d'appareil (20) et **en ce que** le premier élément d'appareil (10) et le deuxième élément d'appareil (20) et/ou le deuxième élément d'appareil (20) et le troisième élément d'appareil (30) sont réalisés de manière à ce qu'il soit possible d'échanger des données entre ceux-ci de manière unidirectionnelle ou bidirectionnelle, le deuxième élément d'appareil (20) comportant une mémoire dans laquelle on mémorise des données de traitement concernant le traitement du patient et/ou des données concernant la poche ou d'autres récipients insérés dans le deuxième élément d'appareil (20) et/ou le troisième élément d'appareil (30).

2. Système de traitement médical selon la revendication 1, **caractérisé en ce que** le premier élément d'appareil (10) et/ou le troisième élément d'appareil (30) est ou sont conçus comme des éléments immobiles ou en grande partie immobiles.

3. Système de traitement médical selon la revendication 1 ou 2, **caractérisé en ce que** le procédé de dialyse est une hémodialyse ou une dialyse péritonéale.

4. Système de traitement médical selon l'une quelconque des revendications précédentes, **caractérisé en ce qu'**au moins l'un des éléments d'appareil - de préférence plusieurs ou tous les éléments d'appareil du système de traitement médical - est conçu de telle sorte que les transmissions de données et/ou de l'énergie sont effectuées sans fil ou avec fil entre deux éléments d'appareil.

5. Système de traitement médical selon la revendication 4, **caractérisé en ce qu'**au moins l'un des éléments d'appareil - de préférence plusieurs ou tous les éléments d'appareil - est ou sont conçu(s) de telle sorte que la transmission de données et d'énergie a lieu simultanément et/ou au moyen du même dispositif.

6. Système de traitement médical selon l'une quelconque des revendications précédentes, **caractérisé en ce que** le deuxième élément d'appareil (20) possède une ou plusieurs mémoires non-volatiles pour le stockage de données.

7. Système de traitement médical selon l'une quelconque des revendications précédentes, **caractérisé en ce que** l'approvisionnement énergétique du deuxième élément d'appareil (20) est conçue de telle sorte qu'elle permet, en cas de panne de l'approvisionnement énergétique du premier élément d'appareil (10) ou du troisième élément d'appareil (30), d'effectuer un approvisionnement énergétique de secours pour le premier élément d'appareil (10) ou le troisième élément d'appareil (30) au moyen du deuxième élément d'appareil (20).

8. Système de traitement médical selon l'une quelconque des revendications précédentes, **caractérisé en ce que** des moyens sont prévus qui sont conçus de telle sorte que des données stockées dans le deuxième élément d'appareil (20) sont transmises à celui-ci ou reçues par celui-ci en approchant le premier élément d'appareil (10) et/ou le troisième élément d'appareil (30).

9. Système de traitement médical selon l'une quelconque des revendications précédentes, **caractérisé en ce que** des moyens de contrôle sont prévus qui sont conçus de telle sorte que les données transmises au côté du deuxième élément d'appareil (20) au premier élément d'appareil (10) ou au troisième élément d'appareil (30) sont soumises à un contrôle, dans lequel il est de préférence prévu, que des moyens de décision sont prévus qui sont conçus de telle sorte, qu'une décision est faite à l'aide du résultat de contrôle effectué dans les moyens de contrôle, si un traitement à l'aide du résultat du contrôle est effectué dans les moyens de contrôle ou pas.

10. Système de traitement médical selon l'une quelconque des revendications précédentes, **caractérisé en ce que** le matériel jetable est doté de marquages lisibles, dans lequel il est de préférence prévu que le marquage est une matrice en deux dimensions, un code à barres ou une étiquette RFID.

11. Système de traitement médical selon l'une quelconque des revendications précédentes, **caractérisé en ce que** le premier élément d'appareil (10) et/ou le deuxième élément d'appareil (20) et/ou le troisième élément d'appareil (30) est doté d'identifiants, de préférence des étiquettes électroniques, au moyen desquels les éléments d'appareil eux-mêmes et/ou l'appartenance d'un élément d'appareil à un autre élément d'appareil peuvent être identifiés.

12. Système de traitement médical selon l'une quelconque des revendications précédentes, **caractérisé en ce que** le premier élément d'appareil (10) et/ou le deuxième élément d'appareil (20) et/ou le troisième élément d'appareil (30) est ou sont dotés d'un ou de plusieurs dispositifs d'affichage et/ou de saisie, en particulier d'affichages, dans lequel il est de préférence prévu que l'affichages est un écran TFT (LCD), OLED ou papier électronique.

13. Système de traitement médical selon l'une quelconque des revendications précédentes, **caractérisé en ce que** le deuxième élément d'appareil (20) est doté d'au moins un réservoir pour recueillir un liquide de traitement frais et/ou usé et/ou un liquide de substitution.

14. Système de traitement médical selon la revendication 13, **caractérisé en ce que** le premier élément d'appareil (10) et/ou le troisième élément d'appareil (30) dispos(ent) de moyens à l'aide desquels le réservoir du deuxième élément d'appareil (20) peut être rempli et/ou vidé.
